# Europäisches Patentamt
# European Patent Office
# Office européen des brevets

(11) Veröffentlichungsnummer : **0 075 262**
**B1**

(12) **EUROPÄISCHE PATENTSCHRIFT**

(45) Veröffentlichungstag der Patentschrift :
03.12.86

(51) Int. Cl.⁴ : **C 12 P 19/30, C 12 N 11/08**

(21) Anmeldenummer : **82108494.4**

(22) Anmeldetag : **15.09.82**

(54) Verfahren zur Herstellung von 5'-Ribonucleotiden.

(30) Priorität : **17.09.81 DE 3136940**

(43) Veröffentlichungstag der Anmeldung :
**30.03.83 Patentblatt 83/13**

(45) Bekanntmachung des Hinweises auf die Patenterteilung : **03.12.86 Patentblatt 86/49**

(84) Benannte Vertragsstaaten :
**AT BE CH DE FR GB IT LI NL SE**

(56) Entgegenhaltungen :
**CHEMICAL ABSTRACTS, Band 87, Nr. 1, 4. Juli 1977,
Seite 180, Nr. 2035k, Columbus, Ohio, USA
CHEMICAL ABSTRACTS, Band 83, Nr. 25, 22. Dezember 1975, Seite 156, Nr. 203288m, Columbus, Ohio,
USA; V.P. VARLAMOV et al.: "Immobilized exonuclease A5 and its use for preparation of 5'-nucleoti-
des"
CHEMICAL ABSTRACTS, Band 94, Nr. 13, 30. März
1981, Seite 577, Nr. 101302w, Columbus, Ohio, USA;
CHUNG-I YUAN et al.: "Use of immobilized 5'-phos-
phodiesterase in industrial production of 5'-nucleoti-
des"
CHEMICAL ABSTRACTS, Band 84, Nr. 19, 10. Mai
1976, Seite 207, Nr. 132168e, Columbus, Ohio, USA;
V.K. KAGRAMANOVA et al.: "Use of immobilized
snake venom phosphodiesterase for the analysis of
the nucleotide composition of oligodeoxyribonucleotides"
CHEMICAL ABSTRACTS, Band 82, Nr. 11, 17. März
1975, Seite 174, Nr. 69823f, Columbus, Ohio, USA;
YU.A. BERLIN et al.: "Hydrolysis of oligodeoxyribonucleotides by phosphohydrolases immobilized on cellulose"
ENZYME ENG., Band 3, 1975, Seiten 469-475; H.
SAMEJIMA et al.: "Production of 5'-mononucleotides
using immobilized 5'-phosphodiesterase and 5'-AMP
deaminase"**

(73) Patentinhaber : **HOECHST AKTIENGESELLSCHAFT
Postfach 80 03 20
D-6230 Frankfurt am Main 80 (DE)**

(72) Erfinder : **Keller, Reinhold, Dr.
Wiesenweg 5
D-6232 Bad Soden am Taunus (DE)**
Erfinder : **Schlingmann, Merten, Dr.
Schneidhainer Strasse 32a
D-6240 Königstein/Taunus (DE)**

Jouve, 18, rue St-Denis, 75001 Paris, France

EP 0 075 262 B1

**Beschreibung**

Gegenstand der Erfindung ist ein Verfahren zur Herstellung von 5'-Ribonucleotiden aus einem Gemisch von Rohnucleinsäuren, wie es z. B. in bekannter Weise aus Mikroorganismen gewonnen werden kann. In derartigen Rohnucleinsäuren liegen neben anderen Stoffen Ribonucleinsäure (RNS) und Desoxyribonucleinsäure (DNS) vor.

5'-Ribonucleotide dienen als Ausgangsstoffe zur Herstellung von Lebensmittelzusatzstoffen und für Arzneimittel. Bekannt ist ihre Herstellung durch enzymatische Hydrolyse von RNS. Das hierzu verwendete Enzym 5'-Phosphodiesterase hydrolysiert jedoch neben RNS gleichzeitig auch DNS, so daß neben den gewünschten 5'-Ribonucleotiden als Nebenprodukte auch 5'-Desoxyribonucleotide entstehen. Diese Nebenprodukte sind von den 5'-Ribonucleotiden nur sehr schwierig abzutrennen. Deshalb ist es zur Herstellung von reinen 5'-Ribonucleotiden erforderlich, schon von einer RNS auszugehen, die praktisch frei von DNS ist.

Zur Herstellung von reiner RNS sind nun schon eine Reihe verschiedener Verfahren bekanntgeworden. Hierzu gehört die selektive Ausfällung von RNS durch Erhitzen und anschließende Säurebehandlung, wie es in der japanischen Patentanmeldung Sho-53-20 493 beschrieben ist. Ein anderes Verfahren zur Reinigung von RNS ist in der japanischen Patentanmeldung Sho 54-55 791 beschrieben, bei dem die RNS durch Säurefällung in Gegenwart zweiwertiger Kationen gewonnen wird.

Bei allen diesen bekannten Verfahren ist es jedoch nachteilig, daß die DNS durch eine Hitze- und Säurebehandlung zersetzt wird. Dabei geht auch ein beträchtlicher Anteil der RNS verloren. Außerdem läßt sich die RNS nur durch Zentrifugieren isolieren, was für ein industrielles Verfahren mit erheblichen Nachteilen verbunden ist.

Es stellt sich deshalb die Aufgabe, ein Verfahren zur Herstellung von 5'-Ribonucleotiden zu finden, welches eine Vorreinigung der RNS und damit eine Zerstörung der DNS nicht erforderlich macht. Gelöst wurde diese Aufgabe erfindungsgemäß dadurch, daß die Reaktionsfähigkeit des zur Hydrolyse benötigten Enzyms, der 5'-Phosphodiesterase, durch eine geeignete Immobilisierung hochselektiv so verändert wird, daß eine Hydrolyse der DNS nicht mehr stattfinden kann.

Erfindungsgegenstand ist deshalb ein Verfahren zur Herstellung von 5'-Ribonucleotiden, das dadurch gekennzeichnet ist, daß man in einer Lösung von Rohnucleinsäuren, die neben Ribonucleinsäure mit einem Molekulargewicht signifikant unter 100 000 auch Desoxyribonucleinsäure mit einem Molekulargewicht von über 100 000 enthält, mit einer an einem polymeren Träger immobilisierten 5'-Phosphodiesterase selektiv die Ribonucleinsäure hydrolysiert.

Es war überraschend, daß diese Reaktion mit immobilisierter 5'-Phosphodiesterase so hochselektiv verläuft, da das Enzym in freier, nativer Form mit ungefähr gleicher Geschwindigkeit RNS und DNS spaltet [M. Fugimoto, A. Kuninaka, H. Yoshino, Agr. Biol. Chem. 38, 1555 (1974)] und da das Enzym bereits nach mehreren Verfahren immobilisiert wurde, ohne daß etwas über die selektive Hydrolyse von RNS bekanntgeworden ist [S. Noguchi, G. Shimura, K. Kimura, H. Sanujima, Journal of Solid-Phase Biochemistry 1, 105 (1976)].

Das Enzym wird durch Ausbildung einer kovalenten Bindung zwischen Enzym und polymerem Träger immobilisiert. Als Enzymträger kommen polymere, poröse Träger wie Cellulosen, z. B. DEAE- Cellulosen (= Diethylaminoethan-Cellulosen), CM-Cellulosen (= Carboxymethyl-Cellulosen), modifizierte Polyacrylamidgele mit Amino- oder Hydroxylgruppen oder verschiedene organische Copolymerisate aus Acrylamid, Methacrylaten, Methacrylsäure-Epoxypropylester, Methacrylamid und Maleinsäureanhydrid in Frage, wie sie in der DE-OS 2 215 539 (US-PSn 3 910 825 und 4 044 196) und der DE-AS 2 732 301 (US-PS 4 247 643) beschrieben sind.

Bevorzugt wählt man als polymeren Träger einen makroporösen Träger aus, der Kanäle mit einem Durchmesser bis zu 100 nm und ein Porenvolumen von etwa 2 bis 3, insbesondere etwa 2,5 ml/g aufweist.

Als besonders guter Träger für die 5'-Phosphodiesterase in dem erfindungsgemäßen Verfahren hat sich der unter dem Handelsnamen (R)Eupergit C geführte Epoxidträger erwiesen, der ein Copolymerisat aus Glycidylmethacrylat, Allylglycidylether, Methacrylamid und Methylen-bis-methacrylamid ist. Solche Träger sind in der DE-OS 2 722 751 (US-PSn 4 190 713 und 4 208 309 beschrieben.

Das Enzym wird mit dem polymeren Träger unter Bedingungen gekuppelt, bei denen die Stabilität des Enzyms nicht leidet. Der Erfolg der Kupplung kann durch die Messung der enzymatischen Aktivität am Polymeren und im Waschwasser bestimmt werden.

Das trägergebundene Enzym wird zur Durchführung des erfindungsgemäßen Verfahrens vorzugsweise in eine Säule gefüllt, durch die man die zu hydrolysierende Lösung der Rohnucleinsäuren in Gegenwart eines Puffersystems durchfließen läßt. Die Durchflußgeschwindigkeit wird dabei so eingestellt, daß gerade eben ein 100 %iger Umsatz der RNS erfolgt.

Die eingesetzte Nucleinsäurelösung wird bevorzugt aus Mikroorganismen durch Extraktion der Lipide mit Ammoniak und niederen Alkanolen und anschließende Aufarbeitung der wäßrigen Waschphase gewonnen (US-PS 4 206 243). Diese Rohnucleinsäuren enthalten eine DNS vom Molgewicht > 100 000, bevorzugt > 200 000, und RNS mit Molgewichten von signifikant < 100 000, insbesondere von 10 000 bis 50 000.

Die erfindungsgemäß zum Einsatz kommende immobilisierte 5'-Phosphodiesterase hat nicht nur eine im Vergleich zur löslichen 5'-Phosphodiesterase überraschend hohe Selektivität von 100 % gegenüber RNS, sondern kann wegen ihrer großen Haltbarkeit auch über eine längere Zeit kontinuierlich verwendet werden. Es ist z. B. möglich, über einen Zeitraum von 10 Monaten die durchlaufenden Ribonucleinsäuremengen vollständig zu den 5'-Ribonucleotiden aufzuspalten.

Das hierbei gewonnene Hydrolysat enthält neben den 5'-Ribonucleotiden und intakter DNS auch noch Verunreinigungen von Proteinen und Fermentationssalzen, die nach an sich bekannten Reinigungsverfahren abgetrennt werden können. Bei der Isolierung von 5'-Ribonucleotiden geschieht dies durch ein Ionenaustauschverfahren oder durch ein Verfahren der Adsorptionschromatographie. Zur Isolierung und Reinigung der DNS hat sich ein bekanntes pH-Fällungsverfahren bewährt. Dabei ist es unwesentlich, welche Isolierung zuerst vorgenommen wird.

Nach dem erfindungsgemäßen Verfahren kann man auch im industriellen Maßstab große Mengen von Rohnucleinsäuren selektiv über längere Zeiträume enzymatisch aufspalten und dabei sowohl reine 5'-Ribonucleotide als auch hochmolekulare DNS gewinnen.

Die durch die Hydrolyse der Rohnucleinsäuren gewonnene Lösung, die unter anderem auch das 5'-Adenosinmonophosphat enthält, kann, ohne daß dazu die vorherige Abtrennung der DNS erforderlich ist, direkt zur Gewinnung des als Geschmacksverstärker einsetzbaren 5'-Inosinmonophosphats verwendet werden. Hierzu wird die das 5'-Adenosinmonophosphat enthaltende Lösung über eine Säule gegeben, die auf einem Träger immobilisierte 5'-Desaminase enthält. Hierbei tritt eine Desaminierung des 5'-Adenosinmonophosphats ein.

Das erfindungsgemäße Verfahren wird durch die folgenden Beispiele erläutert :

### Beispiel 1

2 g 5'-Phosphodiesterase (Nuclease $Rp_1$ der Fa. Amano) werden in 80 ml 1 m Phosphat-Puffer pH 7,8 gelöst. Die Lösung wird auf 20 g Epoxid-Träger (Eupergit C) gegeben, leicht aufgerührt und 3 Tage bei Raumtemperatur stehen gelassen. Das Polymerharz wird abfiltriert und einem Waschprozeß mit den folgenden Flüssigkeiten unterworfen :

1. bidest. Wasser
2. 0,1 m $NaHCO_3$
3. 0,5 m NaCl
4. bidest. Wasser
5. 1 mmolare HCl
6. bidest. Wasser

Das gewaschene Harz wird in 0,05 m Acetat-Puffer vom pH 4,5 aufgenommen und in eine Säule gefüllt. Zur Messung der Aktivität der immobilisierten 5'-Phosphodiesterase wird eine Substrat-Lösung, bestehend aus 4 % RNS und 0,1 mmolarem Zinksulfat in 0,05 m Acetatpuffer vom pH 4,5 mit einer Durchflußgeschwindigkeit (SV) 20-30 $h^{-1}$ bei 55 °C durch die Säule gepumpt. Die Menge der entstehenden Mononucleotide wird photometrisch oder mittels Hochdruck-Flüssigkeitschromatographie ermittelt.

### Beispiel 2

Eine wäßrige Lösung mit 0,9 % (Gew./Vol) Rohnucleinsäure (RNS/DNS 4 : 1) wird mit Eisessig auf pH 4,5 eingestellt und bei einer Temperatur von 55 °C durch eine Säule mit der gemäß Beispiel 1 immobilisierten 5'-Phosphodiesterase fließen gelassen. Die Durchflußgeschwindigkeit der Säule wird dabei so eingestellt, daß gerade ein 100 %iger Umsatz der RNS erfolgt. Zur Isolierung der 5'-Ribonucleotide wird die abgebaute Nucleinsäurelösung über eine Säule gepumpt, die mit einem schwach basischen Ionenaustauscher (Acetat-Form) beladen ist, wo die 5'-Ribonucleotide zur Adsorption gebracht werden. Zur Reinigung und Isolierung der DNS wird das Eluat der Säule mittels Hohlfaser-Membranen aufkonzentriert und gegen entsalztes Wasser dialysiert. Dabei können Moleküle mit Molekulargewichten unter 50 000 die Membran passieren. Die DNS wird durch Einstellen des pH-Wertes der Lösung auf pH 2,0 ausgefällt und nach dem Trocknen in Form eines weißen Pulvers isoliert. Die Trennung und Isolierung der vier verschiedenen 5'-Ribonucleotide erfolgt auf bekannte Weise durch selektive Desorption mit Acetat-Lösungen steigender Konzentration.

### Beispiel 3

Eine wäßrige Lösung von 3 % (Gew./Vol) Rohnucleinsäure (RNS/DNS 4 : 1) wird mit Eisessig auf pH 4,5 eingestellt und bei einer Temperatur von 55 °C durch eine Säule mit der nach Beispiel 1 immobilisierten 5'-Phosphodiesterase fließen gelassen. Die Durchflußgeschwindigkeit der Säule wird dabei so eingestellt, daß gerade ein 100 %iger Umsatz der RNS erfolgt. In der so erhaltenen Lösung ist unter anderem auch das 5'-Adenosinmonophosphat enthalten. Um hieraus 5'-Inosinmonophosphat herzustellen, wird die Lösung über eine zweite Säule mit immobilisierter 5'-Desaminase gegeben. Das Eluat der Säulen wird mit den in Beispiel 2 beschriebenen Hohlfasermembranen kontinuierlich dialysiert. Das Permeat wird mit konzentrierter Salzsäure auf pH 1,5 bis 2,0 eingestellt und über eine Säule, gefüllt mit granulierter Aktivkohle, gepumpt, wobei die 5'-Ribonucleotide zur Adsorption gebracht werden. Aus dem Retenat wird durch Einstellen des pH auf 2,0 die DNS ausgefällt und anschließend getrocknet. Die Isolierung und Trennung der verschiedenen 5'-Ribonucleotide erfolgt auf bekannte Weise durch selektive Desorption mit alkalischen, wäßrigen Methanollösungen.

### Beispiel 4

Eine wäßrige Lösung von 2 % (Gew./Vol) Rohnucleinsäure (RNS/DNS 1 : 1) wird wie in Beispiel 2 vorbehandelt und zum Abbau über die Säule mit immobilisierter 5'-Phosphodiesterase fließen gelassen. Das Eluat wird mit NaOH auf pH 8,5 eingestellt und zum Zwecke der Adsorption der Nucleotide über eine Säule, beladen mit stark basischem Anionenaustauscher IRA 400 (Chlorid-Form), fließen gelassen. Das Eluat der Ionenaustauschersäule wird mit den in Beispiel 2 genannten Hohlfasermembranen aufkonzentriert und mit konzentrierter Salzsäure auf pH 2,0 eingestellt. Die ausgefallene DNS wird abfiltriert und getrocknet. Nach kräftigem Waschen der Ionenaustauschersäule mit entsalztem Wasser werden die einzelnen Nucleotide in hochreiner Form mit Lösungen steigender Chlorid-Konzentration desorbiert und zur Kristallisation gebracht.

**Patentansprüche**

1. Verfahren zur Herstellung von 5'-Ribonucleotiden, dadurch gekennzeichnet, daß man in einer Lösung von Rohnucleinsäuren, die neben Ribonucleinsäure mit einem Molekulargewicht signifikant unter 100 000 auch Desoxyribonucleinsäure mit einem Molekulargewicht von über 100 000 enthält, mit einer an einem polymeren Träger immobilisierten 5'-Phosphodiesterase selektiv die Ribonucleinsäure hydrolysiert.

2. Verfahren nach Anspruch 1, dadurch gekennzeichnet, daß man als polymeren Träger einen makroporösen Träger auswählt, der Kanäle mit einem Durchmesser bis zu 100 nm und ein Porenvolumen von etwa 2-3 ml/g aufweist.

3. Verfahren nach Anspruch 2, dadurch gekennzeichnet, daß der polymere Träger ein Porenvolumen von etwa 2,5 ml/g aufweist.

4. Verfahren einem oder mehreren der Ansprüche 1 bis 3, dadurch gekennzeichnet, daß der Träger ein Copolymerisat aus Glycidylmethacrylat, Allylglycidylether, Methacrylamid und Methylen-bis-methacrylamid ist.

5. Verfahren nach einem oder mehreren der Ansprüche 1 bis 4, dadurch gekennzeichnet, daß die Rohnucleinsäurelösung aus Mikroorganismen durch Extraktion der Lipide mit Ammoniak und niederen Alkanolen und anschließende wäßrige Extraktion erhalten wurde.

6. Verfahren nach einem oder mehreren der Ansprüche 1 bis 5, dadurch gekennzeichnet, daß die Rohnucleinsäure DNS vom Molgewicht über 200 000 und RNS mit Molgewichten von 10 000 bis 50 000 enthält.

7. Verfahren nach einem oder mehreren der Ansprüche 1 bis 6, dadurch gekennzeichnet, daß aus dem Hydrolysat die 5'-Ribonucleotide mit Hilfe von Ionenaustauschern isoliert werden.

8. Verfahren nach einem oder mehreren der Ansprüche 1 bis 6, dadurch gekennzeichnet, daß aus dem Hydrolysat die 5'-Ribonucleotide durch Adsorptionschromatographie isoliert werden.

9. Verwendung des nach einem oder mehreren der Ansprüche 1 bis 8 erhaltenen Hydrolysats zur Herstellung von 5'-Inosinmonophosphat, dadurch gekennzeichnet, daß man aus dem darin enthaltenen 5'-Adenosinmonophosphat mit einer an einem Träger fixierten 5'-Desaminase Ammoniak abspaltet.

**Claims**

1. A process for the preparation of 5'-ribonucleotides, characterized by hydrolyzing selectively ribonucleic acid in a solution of crude nucleic acids, containing desoxyribonucleic acid having a molecular weight higher than 100 000 besides ribonucleic acid having a molecular weight significantly lower than 100 000, with a 5'-phosphodiesterase immobilized to a polymer carrier.

2. The process as claimed in Claim 1, which comprises chosing as polymer carrier a macroporous carrier containing channels having a diameter of up to 100 nm and a pore volume of about 2 to 3 ml/g.

3. The process as claimed in Claim 2, wherein the polymer has a pore volume of about 2.5 ml/g.

4. The process as claimed in one or more of Claims 1 to 3, wherein the carrier is a copolymer of glycidyl methacrylate, allylglycidyl ether, methacrylamide and methylene-bis-methacrylamide.

5. The process as claimed in one or more of Claims 1 to 4, which comprises obtaining the crude nucleic acid solution from microorganisms by extraction of the lipids with ammonia and lower alcohols and subsequent aqueous extraction.

6. The process as claimed in one or more of Claims 1 to 5, wherein the crude nucleic acid contains DNA having a molecular weight greater than 200,000, and RNA having a molecular weight of from 10,000 to 50,000.

7. The process as claimed in one or more of claims 1 to 6 which comprises isolating the 5'-ribonucleotides from the hydrolysate by means of ion exchangers.

8. The process as claimed in one or more of Claims 1 to 6, which comprises isolating the 5'-ribonucleotides from the hydrolysate by means of adsorption chromatography.

9. Use of the hydrolysate obtained as claimed in one or more of Claims 1 to 8 for obtaining 5'-inosine monophosphate, which comprises splitting off ammonia from the 5-adenosine monophosphate contained in the hydrolysate by means of 5'-desaminase fixed on a carrier.

**Revendications**

1. Procédé de préparation de 5'-ribonucléotides, caractérisé en ce que, dans une solution d'acides nucléiques bruts qui, en dehors de l'acide ribonucléique ayant une masse moléculaire bien inférieure à 100 000, contient également de l'acide désoxyribonucléique ayant une masse moléculaire supérieure à 100 000, on hydrolyse sélectivement l'acide ribonucléique avec une 5'-phosphodiestérase immobilisée sur un support polymère.

2. Procédé selon la revendication 1, caractérisé en ce que, comme support polymère, on choisit un support macroporeux qui possède des canaux ayant un diamètre maximal de 100 nm et un volume de pores d'environ 2 à 3 ml/g.

3. Procédé selon la revendication 2, caractérisé en ce que le support polymère a un volume de pores d'environ 2,5 ml/g.

4. Procédé selon une ou plusieurs des revendications 1 à 3, caractérisé en ce que le support est un copolymère de méthacrylate de glycidyle, d'éther allylglycidylique, de méthacrylamide et de méthylène-bis-méthacrylamide.

5. Procédé selon une ou plusieurs des revendications 1 à 4, caractérisé en ce que la solution d'acide nucléique brut est obtenu à partir de micro-organismes par extraction des lipides avec de l'ammoniac et des alcanols inférieurs, puis par extraction aqueuse.

6. Procédé selon une ou plusieurs des revendications 1 à 5, caractérisé en ce que l'acide nucléique brut contient de l'ADN ayant une masse moléculaire supérieure à 200 000 et de l'ARN ayant des masses moléculaires de 10 000 à 50 000.

7. Procédé selon une ou plusieurs des revendications 1 à 6, caractérisé en ce qu'on isole les 5'-ribonucléotides à partir de l'hydrolysat au moyen d'échangeurs d'ions.

8. Procédé selon une ou plusieurs des revendications 1 à 6, caractérisé en ce qu'on isole les 5'-ribonucléotides à partir de l'hydrolysat par chromatographie d'adsorption.

9. Utilisation de l'hydrolysat obtenu selon une ou plusieurs des revendications 1 à 8, pour la préparation de monophosphate de 5'-inosine, caractérisée en ce qu'à partir du monophosphate de 5'-adénosine contenu dans l'hydrolysat, on élimine l'ammoniac avec une 5'-désaminase fixée sur un support.